(19) Europäisches Patentamt
European Patent Office
Office européen des brevets

(11) EP 0 666 925 B1

(12) **EUROPEAN PATENT SPECIFICATION**

(45) Date of publication and mention of the grant of the patent:
**07.01.1999 Bulletin 1999/01**

(21) Application number: **93922931.6**

(22) Date of filing: **11.10.1993**

(51) Int Cl.[6]: **C12P 7/64**, C11C 3/10, A23G 1/00

(86) International application number:
**PCT/EP93/02788**

(87) International publication number:
**WO 94/10326 (11.05.1994 Gazette 1994/11)**

(54) **ENZYMIC TRIGLYCERIDE CONVERSION**

ENZYMATISCHES TRIGLYCERID-UMSETZVERFAHREN

CONVERSION ENZYMATIQUE DE TRIGLYCERIDES

(84) Designated Contracting States:
**AT BE CH DE DK ES FR GB GR IE IT LI NL PT SE**

(30) Priority: **29.10.1992 EP 92309904**

(43) Date of publication of application:
**16.08.1995 Bulletin 1995/33**

(73) Proprietor: **LODERS CROKLAAN B.V.**
**NL-1521 AX Wormerveer (NL)**

(72) Inventors:
- **CHANDLER, Ian, Christopher, Unilever Research Sharnbrook, Bedford MK44 1LQ (GB)**
- **QUINLAN, Paul, Thomas, Unilever Research Sharnbrook, Bedford MK44 1LQ (GB)**

(74) Representative:
**Rots, Maria Johanna Francisca et al**
**Unilever N.V.,**
**Patent Division,**
**P.O. Box 137**
**3130 AC Vlaardingen (NL)**

(56) References cited:
**GB-A- 1 577 933**

- **BIOCHIMICA ET BIOPHYSICA ACTA vol. 792, no. 3 , 7 March 1984 pages 254 - 269 M. BUGAUT ET AL. 'An examination of the stereochemical course of acylation of 2-monoacylglycerols by rat intestinal villus cells using [2H3]palmitic acid'**
- **PROCEEDINGS OF THE WORLD CONFERENCE ON BIOTECHNOLOGY FOR THE FATS AND OILS INDUSTRY, 1987 1988 pages 328 - 329 RICARDO SCHUCH ET AL. 'Interesterification of lipids by an sn-1,3-specific triacylglycerol lipase'**
- **CHEMISTRY AND PHYSICS OF LIPIDS vol. 61, no. 2 , April 1992 pages 193 - 198 DETLEF MEUSEL ET AL. 'Stereoselectivity of lipases: esterification reactions of octadecylglycerol' cited in the application**
- **CHEMICAL ABSTRACTS, vol. 115, no. 17, 28 October 1991, Columbus, Ohio, US; abstract no. 181880, YAMAGUCHI, KOTARO ET AL. 'Fats and oils containing 1-saturated-2-unsaturated fatty acid triglycerides and soft chocolates containig them' page 790 ;column R ; & JP,A,3 043 042 (FUJI OIL CO., LTD.) 25 February 1991**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 666 925 B1

## Description

Although it has been recognized in the prior art that asymmetrical triglycerides of the type $R_1$ $R_2$ $R_3$ or $R_1$ $R_1$ $R_3$ can exist in different stereo-isomeric forms, it was so far thought that known POS-samples, synthesized by enzymatic interesterification were racemic mixtures (cf. Arishima c.s., JAOCS, 68, no. 10 (1991), p. 710-715).
In fact, above statement was a confirmation of the disclosures in EP 93 602 B-1, wherein on page 2, lines 52-55 it is stated that the stereo specifity of Rh. arrhizus lipase in enzymic conversions was investigated, but was found to be non-existent.

The above statements were striking, as it is also known from literature that enzymes, including lipases can be used in processes for the production of products enriched in a specific enantiomer. E.g. in Biocatalysis, 1990, volume 3, p. 307-317 Jensen c.s. discloses that certain lipases are selective for specific alcohols or fatty acids. In J. Am. Chem. Soc. (1985) no. 107, p. 7072-7076 Kirchner c.s. disclose that yeast lipase and porcine pancreatic lipase catalyze the reactions of esterification and transesterification in a stereo-selective manner. According to this technique optically active alcohols, carboxylic acids and their esters have been prepared. Ching-Shih Chen and C.J. Sih described in Angew. Chem., Int. Ed. Engl. 28 (1989), p. 695-707, which basic principles govern lipase-catalyzed enantio-selective esterification and transesterification for the preparation of optically active acids and alcohols.
Lipase-catalyzed enantio-selective hydrolysis is the subject of the paper of Mitsuda c.s. in Agric. Biol. Chem. 54 (11), 1990, p. 2907-2912. A similar disclosure can be found in Journ. Biol. Chem. 265 (1990), 20271 in a paper from Rogalska, Ransac and Verger.

In a recent publication of Meusel et al. in Chem. and Phys. of Lipids 61 (1992), p. 193-198 it is disclosed a.o. that Rhizomucor miehei lipase displays a very slight stereoselectivity for the Sn-1 position in the enzymic preparation of 1-o-octadecylglycerol.

However, from none of the above cited prior art indications can be derived how to come to an enzymic process for the preparation of optically active triglyceride compositions. Such a process would create useful new fat compositions with physical properties that will be different from the known mesomeric compositions.

E.g. we found that chiral StOP transformed much faster to higher polymorphic forms than racemic StOP. Further, chiral StOP displayed a crystallisation behaviour which made it a good match for cocoa butter. Racemic StOP nucleated and crystallised more slowly. Therefore, chiral StOP is better applicable in chocolate compositions than racemic StOP (cf. example VI).

Triglycerides, wherein the 1 and 3-positions are occupied by different fatty acids exist in optically active forms. When a Fisher projection is made of such an optically active triglyceride with the SN-1 position to the top, the SN-3 position to the bottom and the secondary hydroxyl group to the left two stereo-isomeric forms are possible, as illustrated below:

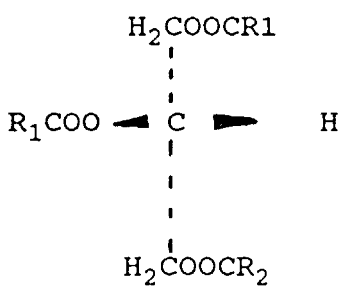

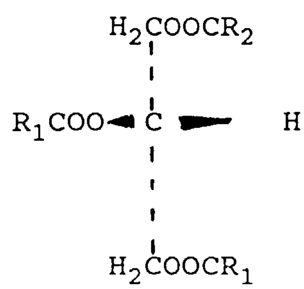

when $R_1$= palmityl and $R_2$= oleyl, one isomer is indicated as PPO, the other isomer as OPP.

Christie et al. (1991) J. Am. Oil Chem. Soc. 68, 695-701 have disclosed a method, based on high performance liquid chromatography for the resolution of the different diastereomeric isomers. This method gives us the opportunity to determine both the Sn-1 and Sn-3 composition of the triglyceride and thus to distinguish the above PPO isomer from the OPP isomer.

We have found a new process that results in the formation of asymmetric triglycerides from which more than one enantiomer exists and which triglyceride compositions are enriched in one or more of the possible enantiomers. According to our process the following requirements are essential:

1. the lipase should be a lipase derived from Mucor species, also known as Zygomucor or Rhizomucor, in particular Mucor miehei, the lipase can either be natural or a modified lipase, wherein the modified lipase is obtained by genetic engineering
2. the water activity of the reaction mixture should be kept below 0.5

3. further conditions, such as temperature, contact times, ratio of reactants, use of a solvent should be selected that result in an overall degree of conversion as defined below of 40-95%, preferably 50-85%.

It is essential that the lipase is derived from Mucor species, also known as Zygomucor or Rhizomucor miehei. The lipase can be used per se, or can be supported on any known support (see e.g. EP 322 213). Preferred supports are organic polymers, such as duolite or accurel. Lipases that can be used, according to above definition of our lipases are:

- natural Mucor miehei, Zygomucor or Rhizomucor miehei lipases
- lipases from another host, capable of producing the same lipases, due to the introduction of an expressable lipase gene into that host
- modified lipases with a similar stereo-specific activity, produced by any suitable host, being the result of expression of a modified lipase gene.

The lipase may be used in a batch reaction or in a packed bed reactor system.

It has been found that the magnitude of the enantiomerenrichment is directly related to the initial water activity. The lower the water activity, the larger the effect (cf. table I). However, the lowerlimit for the water activity is formed by the reaction rate that is acceptable, as the reaction rate is directly related to the water activity. Therefore, the water activity cannot be indefinitely small. Preferred ranges are 0.01-0.4, in particular 0.05-0.3.

Another important factor that must be considered is the overall degree of conversion, which should be controlled to achieve maximum production of the asymmetric triglyceride. The conversion at which this occurs will vary with the ratio of the two reactants. The reaction must not be taken to equilibrium, as this will result in racemisation of the triglyceride species. Therefore, the reaction conditions should be chosen that guarantee that an overall conversion of 40-95%, preferably 50-85% occurs, the conditions that have to be chosen are a.o. the reaction temperature (normally from 40-80°C), the contact time (less than 3 hrs, in particular < 1 hr, and most preferably 15-45 min in a packed bed), the weight ratio of the reactants (as the end product must be asymmetric, it should be avoided to use so much of a reactant that a symmetric product would be formed). Also the application of a solvent and the water activity chosen according to point 2 will play a role in the overall conversion rate.

Partial enzymic triglyceride conversions are known from e.g. EP 34 065 and EP 35 883. Therefore, the conditions for a partial conversion can also be derived from these references.
However, in these references a stereo-specific conversion is not disclosed, as e.g. a Rhizomucor miehei lipase was never applied for the production of an asymmetrical triglyceride.

The principle described above applies to the production of any asymmetric TAG, examples of which could include enantiomers of StOP, PPU, StStU, PStO, PPBu, StStBu, BUU, BLnLn, BMM, AUU, MMO and HHM type TAG's etc. St= stearic acid; P= palmitic; B= behenic; A= arachidic; Bu= butyric; U= unsaturated fatty acid; O= oleic; Ln= linoleic; M= $C_8$-$C_{14}$ sat. fatty acid; H= saturated fatty acid $C_{16}$-$C_{24}$.

Suitable conversions are conversion between the triglycerides of claim 7 and the reactants of claim 8.

In addition to the above findings we have also found another unexpected advantage of our new process. This advantage will be explained by using the drafts of Figures 1 and 2, which by way of example only describe the enzymic reaction between tripalmitin ($P_3$) and oleic acid. In this reaction $P_3$ is converted in a first step into a mixture of PPO and OPP, whereupon in a second step OPO is formed. In those figures a plot is given for the correlation between the amount of triglyceride that is produced depending on the reaction (or contact) time. In figure 1 the theoretical (= calculated) time course of an Sn-1,3 interesterification (for $P_3$ plus oleic acid) is shown. As can be seen from the graph the asymmetric triglyceride (PPO) is produced early on in the reaction and reaches it's equilibrium value of about 48% of the total triglyceride composition. At one point in the reaction the PPO slightly overshoots its equilibrium value, reaching a maximum value of 50% before declining to 48% at equilibrium. In figure 2, however, the actual situation is presented as found for our conversion. It can be seen that early on in the reaction the production of PPO overshoots its equilibrium value, reaching a maximum value of approximately 57% before declining slowly to 48% at equilibrium. Above means that by limiting the contact times a higher yield of the (asymmetric) triglycerides will be obtained compared to the yields obtained at equilibrium. In fact, we have found that the maximum yield obtainable by conversion of trioleoylglycerides with behenic acid (1:3 mole ratio) can even be 59% (while the equilibrium value is 47.5%) (cf. table II). Conversion of tripalmitic triglyceride with ethylbutylrate had an even more striking effect (max. yield: 46%, compared with 16.6% at equilibrium) (cf. table II). Very good yields of the asymmetric triglycerides are also obtained, when triglycerides, containing short chain fatty acids are used. E.g. in the reaction of triacetin with behenic acid the maximum yield of asymmetric triglyceride was 76.4%, compared with 43.2% at equilibrium (table II).

The various examples demonstrate that increased yields of asymmetric TAG's can be obtained under the conditions of the invention with a broad range of triglycerides and fatty acid substrates. A number of examples are summarized in table II.

The process according to the invention can be applied for the production of any type of asymmetric triglyceride

composition and as such the chain length of the fatty acid moieties of the triglycerides and the fatty acid reactants can be $C_1$-$C_{26}$, preferably $C_2$-$C_{22}$, in particular $C_2$-$C_{18}$. In the triglyceride end-products also unsaturated (i.e. both mono- and polyunsaturated) fatty acid moieties can be present. In particular residues from $C_{18:1}$; $C_{18:2}$; $C_{18:3}$, $C_{20:4}$, $C_{20:5}$ and $C_{22:6}$ can be present, including both cis and trans isomers.

Furthermore, the fatty acid reactant described above may be another triglyceride, such that a triglyceride/ triglyceride interesterification is performed. In this situation the degree of conversion is determined by changes in carbon number distribution of the triglycerides, and the optical activity of the resultant triglycerides is determined as described earlier after separation of the triglyceride species by fractionation or chromatographic techniques.

In this application the overall conversion of a reaction between triglyceride and fatty acid moieties is defined as:

$$\text{conversion}=\frac{(FAs - FAi)}{(FAe-FAi)} \times 100\%$$

FAi= initial conc. of fatty acid in TAG
FAe= equilibrium " " " " "
FAs= measured " " " " " " at time t

The water activity is defined as:

$$A_w=\frac{P}{P_o}$$

P= water vapour pressure of sample
$P_o$= water vapour pressure of pure water at same temp.

Enantiomeric excess= (B-A)/(A+B)x100%, where A and B are the concentrations of the two possible enantiomers.

So, the invention also concerns with enzymically made asymmetric triglyceride compositions enriched in at least one of the possible enantiomers. In particular, compositions enriched for more than 10 wt%, more particularly for more than 30 wt% are part of the invention. In these compositions the molar ratios of the possible enantiomers is other than 1.0, preferably more than 1.5, or less than 0.7, in particular more than 3.0 or less than 0.33, more particularly more than 4.0 or less than 0.25. The chiral triglycerides that are preferred comprise mainly POSt/StOP-triglycerides, preferably more than 40 wt%. Chocolates containing such chiral triglycerides are also part of the invention.

Examples:

Example I

The same experimental procedure has been used for all examples. Details are given for a reaction between tripalmitin (PPP) and oleic acid (O) to produce OPP/PPO and OPO.

1 part Tripalmitin
1.048 parts oleic acid (1:3 molar ratio)
0.1-0.5 parts catalyst, pre-equilibrated to a low water activity (< 0.5).
Solvent, if required, preferably hydrophobic e.g. Hexane or Petroleum Ether.

The reaction when taken to equilibrium typically takes 2-3 hours under these conditions and the reaction rate depends on amount of catalyst used and $a_w$.

The Mucor miehei catalyst is pre-equilibrated to a low water activity in controlled humidity atmospheres. This is achieved by keeping the catalyst in a sealed environment containing saturated inorganic salt solutions of known equilibrium humidities for at least 7 days. The saturated solutions used are as follows:

| Salt | Water activity at 20°C |
|---|---|
| Sodium Hydroxide | 0.06 |
| Lithium Chloride | 0.11 |
| Potassium Acetate | 0.23 |

(continued)

| Salt | Water activity at 20°C |
|---|---|
| Magnesium Chloride Hexahydrate | 0.32 |
| Magnesium Nitrate Hexahydrate | 0.55 |
| Sodium Sulphate Decahydrate/Anhydrous Sodium Sulphate mix | 0.77 |
| Potassium Chloride | 0.86 |
| Pure Water | 1.00 |

The effect of initial water activity of the reaction is shown in table I.

Table I

| Effect of initial water activity | | | | |
|---|---|---|---|---|
| INITIAL WATER ACTIVITY | EQUILIBRIUM YIELD OF TAG YXX (wt% of TAG) | MAXIMUM OBTAINED IN REACTION (wt% of TAG) | FAME CONVERS. (%) AT WHICH MAXIMUM OBTAINED | WT% OF EQM. VALUE |
| < 0.01 | 47.7 | 56.3 | 72.2 | 118.0 |
| 0.025 | 47.7 | 54.2 | 82.8 | 113.6 |
| 0.07 | 47.7 | 53.5 | 79.0 | 112.2 |
| 0.17 | 47.7 | 53.2 | 80.7 | 111.5 |
| 0.32 | 47.7 | 51.1 | 78.9 | 107.1 |
| 0.55 | 47.7 | 50.4 | 82.3 | 105.6 |

The lowest water activities (< 0.01) and hence the greatest lipase selectivities, are achieved by drying the catalyst under vacuum for at least 24 hours.

A range of triglycerides and fatty acid substrates were reacted at different molar ratios (as described above). The maximum yield obtained of the asymmetric triglycerides, compared to equilibrium values are listed in table II. Note that the "FAME-conversion value at which maximum obtained" represents the maxiumum observed values in a time course reaction; the optimum conversion may be slightly different from this value.

Table II

| Results for different reaction systems employing *Rhizomucor miehei* lipase | | | | | |
|---|---|---|---|---|---|
| TG/FFA SYSTEM | MOLE RATIO | EQUILIBRIUM YIELD OF TAG YXX (wt % of TAG) | MAXIMUM OBTAINED IN REACTION (wt% of TAG) | FAME CONVERSION (%) AT WHICH MAXIMUM OBTAINED | WT% OF EQM. VALUE |
| PPP/ EtBu | 1:27 | 16.6 | 46.0 | 80.4 | 277.1 |
| PPP/O | 1:1 | 44.9 | 48.0 | 90.5 | 106.9 |
| PPP/O | 1:3 | 47.7 | 56.3 | 69.0 | 118.0 |
| OOO/St | 1:3 | 48.0 | 57.8 | 58.0 | 120.4 |
| OOO/B | 1:3 | 47.4 | 59.1 | 81.4 | 124.6 |
| MMM/O | 1:3 | 47.3 | 56.7 | 72.3 | 119.7 |
| StOSt/P | 1:3 | 48.9 | 57.8 | 71.2 | 118.2 |
| PO-s/ HOSF-acids | 1:3 | 39.3 | 50.6 | 69.6 | 128.7 |
| MMM/B | 1:3 | 45.2 | 63.4 | 68.4 | 140.4 |

Table II (continued)

| Results for different reaction systems employing Rhizomucor miehei lipase | | | | | |
|---|---|---|---|---|---|
| TG/FFA SYSTEM | MOLE RATIO | EQUILIBRIUM YIELD OF TAG YXX (wt % of TAG) | MAXIMUM OBTAINED IN REACTION (wt% of TAG) | FAME CONVERSION (%) AT WHICH MAXIMUM OBTAINED | WT% OF EQM. VALUE |
| PPP/M | 1:3 | 48.7 | 54.1 | 74.6 | 111.0 |
| AcAcAc/ O | 1:3 | 43.6 | 70.0 | 71.3 | 160.5 |
| AcAcAc/ B | 1:3 | 43.2 | 76.4 | 64.0 | 177.1 |
| BuBuBu/ O | 1:3 | 44.5 | 65.8 | 64.5 | 147.8 |
| POP/B | 1:3 | 47.1 | 56.2 | 64.0 | 119.3 |

P= palmitic (= $C_{16}$)
St= stearic (= $C_{18}$)
B= behenic (= $C_{22}$)
O= oleic ($C_{18:1}$)
Bu= Butyric ($C_4$)
EtBu= ethylbutyrate
Ac= Acetic ($C_2$)
M= $C_8$-$C_{14}$
PO-s= Palm oil stearine
HOSF-acids= High oleic sunflower acids

The effect of using various lipase catalyts under conditions of low water activity (< 0.3) and controlled conversion are shown in table III. The results show that only with Rhizomucor miehei lipase the yield of asymmetric triglycerides is increased. The nature of the support (i.e.: accurel versus duolite) was unimportant.

Table III

| All the following reactions have used the PPP/O (Tripalmitin + Oleic acid) 1:3 molar system for comparative purposes | | | | |
|---|---|---|---|---|
| CATALYST USED | EQUILIBRIUM YIELD OF TAG YXX (wt% of TAG) | MAXIMUM OBTAINED IN REACTION (wt% of TAG) | FAME CONVERSION (%) AT WHICH MAXIMUM OBTAINED | WT% OF EQM. VALUE |
| **Rhizomucor miehei** | | | | |
| SP392/Duolite | 47.7 | 56.3 | 69.0 | 118.0 |
| SP282/Duolite | 47.7 | 55.0 | 69.9 | 115.3 |
| Lipozyme/Accurel | 47.7 | 55.4 | 70.3 | 116.1 |
| **Rhizopus** sp. | | | | |
| **R.niveus**/Accurel | 47.7 | 48.1 | 96.0 | 100.8 |
| **R.delemar**/Accurel | 47.7 | 48.2 | 95.2 | 101.0 |
| **R.arrhizus**/Accurel | 47.7 | 49.7 | 86.8 | 104.2 |
| **Humicola Lanuginosa** | | | | |
| on Accurel | 47.7 | 49.2 | 81.4 | 103.1 |
| or on Duolite | 47.7 | 50.0 | 78.1 | 104.9 |

Example II

In accordance with example I POP was converted with stearic acid. The molar acid:oil ratio was 3:1 and the initial water-activity < 0.2. The reaction was stopped at a conversion of 67%. The product of the enzymic conversion displayed the following carbon number distribution:

$C_{50}$: 25.3
$C_{52}$: 55.9
$C_{54}$: 17.3

The sn-1,2 and 3 positions of the triglycerides were determined by the method of Christie et al. described previously with the following result:

| Fame (wt%) | TG | sn-1 | sn-2 | sn-3 |
|---|---|---|---|---|
| 16:0 | 39.0 | 37.1 | 5.4 | 74.6 |
| 18:0 | 29.3 | 60.6 | 3.8 | 23.6 |
| 18:1 | 31.6 | 2.3 | 90.8 | 1.8 |
| TG= triglyceride | | | | |

It can be seen that the palmitic and oleic acid distribution at the sn-1,3 positions is not random and that the triglyceride composition therefore contains unequal proportions of the triglyceride enantiomers. Using the "sn-1 random, sn-2 random, sn-3 random" distribution theory the content of the enantiomers POSt and StOP in this sample is calculated as 7.9% and 41% respectively. The ratio between these two enantiomers (StOP/POSt) therefore is ~ 5.2 and the enantiomeric excess is 68%.

Example III

The conversion was repeated starting now from StOSt and palmitic acid with other conditions as in Example II. The FAME and carbon number distribution of the partially converted (67%) product was:

$C_{50}$: 11.1
$C_{52}$: 55.3
$C_{54}$: 33.4

The sn-1,2 and 3 positions of the triglyceride were determined as before with the following result:

| | TG | sn-1 | sn-2 | sn-3 |
|---|---|---|---|---|
| 16:0 | 26.6 | 55.5 | 10.3 | 14.1 |
| 18:0 | 39.0 | 36.3 | 3.1 | 77.6 |
| 18:1 | 34.4 | 8.2 | 86.6 | 8.4 |

The non-random distribution at the sn-1,3 positions is indicative of the presence of an enantiomeric excess of the asymmetric triglyceride species. The content of POSt and StOP in this sample was calculated as 37.3 and 4.4% respectively, representing an enantiomeric ratio (POSt/StOP) of 8.5 and the enantiomeric excess is 79%.

Example IV

Tripalmitin-rich feed stock and oleic acid (1:3 molar ratio) were reacted in the presence of Rhizomucor miehei lipase as described in Example II. The product of the partial enzymic conversion (69%) was analyzed as above with the following result:

| | TG | sn-1 | sn-2 | sn-3 |
|---|---|---|---|---|
| 16:0 | 72.2 | 63.4 | 58.2 | 95 |
| 18:1 | 27.8 | 36.6 | 41.8 | 5 |

From the above it can be calculated that the OPP and PPO content of this sample is 20.2% and 1.8%, respectively, representing an enantiomeric ratio (OPP/PPO) of 11.2. Likewise, the OOP and and POO content can be calculated as 14.5 and 1.3%, a ratio of 19.3 and the enantiomeric excess is 84%.

Example V

A triglyceride product rich in disaturated triglycerides of the type PPU (where U is oleic or linoleic) were mixed in a 1:1 weight ratio with fatty acids derived from high oleic sunflower and low erucic rape oil. The mixture was partially wetted using a silica pre-column such that the water activity of the feed stream was 0.5. This feed mixture was passed through a column containing 1 kg of Rhizomucor miehei lipase immobilised on duolite (code SP-392 ex Novo) at a temperature of 70°C, and a flow rate between 1 and 1.5 kg/h. An overall conversion (measured as the decrease in palmitate content of the triglyceride) of the final product of 93% was achieved. The fatty acids and partial glycerides were removed, and the resultant triglyceride was fractionated in acetone (6:1 solvent:oil) at -5°C to remove the bulk of the remaining tri- and disaturated triglycerides. The final oleine was analysed to determine the sn-1, 2 and 3 distribution. Results are shown below:

| Fatty acid analysis of triglyceride feed to enzyme column, and resultant product | | | | |
|---|---|---|---|---|
| | Triglyceride total FAME | Feed sn-2 position | Calculated equilibrium after reaction with acids | Triglyceride product |
| 14:0 | 0.8 | 1.2 | 0.5 | 0.6 |
| 16:0 | 67.5 | 91.8 | 46.3 | 47.9 |
| 18:0 | 6.4 | 3.4 | 4.1 | 4.1 |
| 18:1 | 22.1 | 2.8 | 34.5 | 32.5 |
| 18:2w6 | 2.3 | 0.3 | 11.5 | 11.3 |
| 18:3w3 | - | - | 3.0 | 3.0 |

| stereospecific analysis of product oleine fraction | | | | |
|---|---|---|---|---|
| | total TG | sn-1 | sn-2 | sn-3 |
| 16:0 | 34.2 | 12.8 | 77.6 | 12.1 |
| 18:0 | 1.8 | 0.1 | 4.6 | 0.6 |
| 18:1 | 44.4 | 64.7 | 17.6 | 50.9 |
| 18:2w6 | 16.3 | 22.6 | 0 | 26.3 |
| 18:3w3 | 3.3 | -0.3 | 0.3 | 10.0 |

The oleic, linoleic and $\alpha$-linolenic content at the sn-1 and sn-3 positions are different in this triglyceride indicating the presence of unequal proportions of some enantiomers. For example, the content of OPln and lnPO (ln= linoleic acid) in this can be calculated as 13.2% and 8.9% the ratio between these two enantiomers (OPln/lnPO) is 1.48 giving an enantiomeric excess of 19.5%. Furthermore, the calculated content of OPlN (lN= $\alpha$-linolenic) in this sample is 5%, whereas the enantiomer lNPO is surprisingly absent.

Example VI

Chiral StOP-rich fat (chiral CBE) was prepared by reacting pure POP (4.9g) with pure stearic acid (5.0g) in the presence of Rhizomucor miehei catalyst pre-equilibrated to an $a_w$ of 0.1 using the methods and conditions described in Example II.

Racemic StOP-rich fat (racemic CBE) was prepared by blending pure POP (1.59g) with pure StOSt (0.657g) and pure racemic POSt (2.72g).

By carbon number GC the fats had the following compositions (wt%) (in this analysis the positional distribution is not discriminated, thus "StOP"= POSt + StOP + PstO + StPO etc).

| Chiral CBE | Racemic CBE | Cocoa butter |
|---|---|---|
| POP 30.9% | POP 32% | POP 16.6% |

(continued)

| Chiral CBE | Racemic CBE | Cocoa butter |
|---|---|---|
| StOP 53.1%<br>StOSt 12.6%<br>Others 3.4% | StOP+POSt 55%<br>StOSt 13% | StOP+POSt 38.9%<br>StOSt 25.5%<br>others 19% |

with POP/StOP+POSt/StOSt ratios as follows:

| | Chiral CBE | Racemic CBE | Cocoa butter |
|---|---|---|---|
| POP | 32 | 32 | 20.5 |
| StOP+POSt | 55 | 55 | 48 |
| StOSt | 13 | 13 | 31.5 |

The sn-1,2 and 3 positions of the fats were determined as described by Christie et al. with the following result:

| Chiral CBE | | | | |
|---|---|---|---|---|
| FAME (mol%) | TG | sn-1 | sn-2 | sn-3 |
| 16:0 | 40.2 | 44.1 | 0 | 78.2 |
| 18:0 | 25.9 | 55.5 | 0.1 | 20.3 |
| 18:1 | 33.8 | 0 | 99.0 | 0 |

| Racemic CBE | | | | |
|---|---|---|---|---|
| Fame (mol%) | TG | sn-1 | sn-2 | sn-3 |
| 16:0 | 39.6 | 61.3 | 0.1 | 61.3 |
| 18:0 | 25.9 | 38.6 | 0.1 | 38.5 |
| 18:1 | 33.8 | 0 | 99.8 | 0 |

| Cocoa butter | | | | |
|---|---|---|---|---|
| Fame (mol%) | TG | sn-1 | sn-2 | sn-3 |
| 16:0 | 28.4 | 39.0 | 3.6 | 42.6 |
| 18:0 | 35.4 | 52.0 | 0.7 | 53.5 |
| 18:1 | 32.6 | 5.6 | 91.3 | 2.5 |
| 18:2 | 2.8 | 2.3 | 4.7 | 1.4 |

The racemic CBE and cocoa butter have very similar sn-1 and sn-2 analyses, whereas the chiral CBE shows substantial differences at these positions. The calculated StOP and POSt content in this fat is 43.4 and 8.9, respectively, representing an enantiomeric ratio of 4.9 and an enantiomeric excess of 66%.

The fats were seeded by extremely fast cooling to 0°C and melting at 32.5°C. The fats were then crystallised at 20°C and 25°C in comparison with cocoa butter.

The chiral CBE crystallised rapidly in the same epolymorphic form as cocoa butter: the racemic CBE crystallised in a lower polymorphic form at 20°C and very slowly in the normal form (V) polymorphic form at 25°C.

| | Crystallisation (solids/min) 20° C | polymorph | Crystallisation (solids/min) 25°C | polymorph |
|---|---|---|---|---|
| Chiral CBE | 3.8 | β (V) | 2.1 | β (V) |
| Racemic CBE | 2.5 | β' (IV) | 0.3 | β (V) |
| Cocoa butter | 3.6 | β (V) | 1.7 | β (V) |

The fats had the following solids values after crystallisation at 20°C:

| | N20 | N25 | N30 | N32.5 | N35 |
|---|---|---|---|---|---|
| Chiral CBE | 92.6 | 88.9 | 45.6 | 17.0 | 0 |
| Racemic CBE | 91.8 | 68.3 | 30.5 | 15.0 | 0 |
| Cocoa butter | 81.2 | 74.7 | 40.8 | 17.8 | 0.2 |

Therefore, the chiral CBE is a very good replacement for cocoa butter in tempered confectionery chocolate products (despite containing considerably more of the POP triglyceride component and less StOSt than cocoa butter) whereas the racemic CBE will be slow to temper and more prone to bloom on storage.

Example VII

In accordance with example I, 12g POP was reacted with 14.7g behenic acid (in a 1:3 molar ratio) in the presence of 200 ml hexane and 6g Rhizomucor miehei lipase at an initial water activity < 0.2. The reaction was stopped at a conversion of 63.9% and the product triglyceride displayed the following carbon number distribution:

$C_{50}$ 32.52 wt% (POP)
$C_{56}$ 56.23 wt% (BOP+POB)
$C_{62}$ 12.00 wt% (BOB)

The $C_{56}$ value shows a 19.3% increase in weight over the expected equilibrium content (47.1%).

Example VIII

In accordance with example I, 250mg of tripalmitin was reacted with 185.8mg of lauric acid (in a 1:3 molar ratio) in the presence of 5ml of hexane and 125mg of Rhizomucor miehei lipase at an initial water activity < 0.1. The reaction was sampled at intervals and a maximum of the assymetric TAG product LaPP was obtained at a conversion of 74.6%. The carbon number distribution of the triglyceride mix at this point was:

$C_{40}$ 15.6 wt% (LaPLa)
$C_{44}$ 54.1 wt% (LaPP+PPLa)
$C_{48}$ 30.0 wt% (PPP)

The $C_{44}$ value shows a 11% increase in weight over the expected equilibrium content (48.7 wt%) (La= lauric). (AC = Acetic)

Example IX

In accordance with example I, 250mg of tricaprylin (trioctanoin) was reacted with 541.4mg of behenic acid (in a 1: 3 molar ratio) in the presence of 5ml of hexane and 125mg of Rhizomucor miehei lipase at an initial water acitivity < 0.1. The reaction was sampled at intervals and a maximum of the assymetric TAG product BCyCy was obtained at a conversion of 68.4%. The carbon number distribution of the triglyceride mix at this point was:

$C_{24}$ 21.54 wt% (CyCyCy)
$C_{38}$ 63.42 wt% (BCyCy+CyCyB)
$C_{52}$ 15.04 wt% (BCyB)

The $C_{38}$ value shows a 40.4% increase in weight over the expected equilibrium content (45.2%) (Cy=caprylic).

Example X

In accordance with example I, 150mg of triacetin was reacted with 579.5mg of oleic acid (in a 1:3 molar ratio) in the presence of 5ml of hexane and 125mg of Rhizomucor miehei lipase at an initial water activity < 0.1. The reaction was sampled at intervals and a maximum of the assymetric TAG product OAcAc was obtained at a conversion of 71.3%. The carbon number distribution of the triglyceride mix at this point was:

$C_6$ 13.04 wt% (AcAcAc)

$C_{22}$ 69.98 wt% (OacAc+AcAcO)
$C_{38}$ 16.46 wt% (OAcO)

The $C_{22}$ value shows a 60.5% increase in weight over the expected equilibrium content (43.6%). (Ac=Acetic)

Example XI

In accordance with example I, 150mg of triacetin was reacted with 698.6mg of behenic acid (in a 1:3 molar ratio) in the presence of 5ml of hexane and 125mg of Rhizomucor miehei lipase at an initial water activity < 0.1. The reaction was sampled at intervals and a maximum of the assymetric TAG product BAcAc was obtained at a conversion of 64.0%. The carbon number distribution of the triglyceride mix at this point was:

$C_6$ 14.30 wt% (AcAcAc)
$C_{26}$ 76.43 wt% (BAcAc+AcAcB)
$C_{46}$ 9.28 wt% (BAcB)

The $C_{26}$ value shows a 77.1% increase in weight over the expected equilibrium content (43.2%).

## Claims

**1.** Process for the preparation of enzymically converted triglycerides characterized by that asymmetric triglycerides are prepared in which different stereo-isomers exists, which stereo-isomers are present in a molecular ratio other than 1, whereby a suitable triglyceride-starting material is converted with a suitable reactant for the introduction of a required fatty acid moiety into the starting triglyceride, which process is performed by using lipases, derived from Mucor species, also known as Zygomucor or Rhizomucor, in particular Mucor miehei, while the water activity of the reaction mixture is below 0.5 and the conversion is carried out under conditions that give an overall degree of conversion of 40-95%.

**2.** Process according to claim 1, wherein the lipase is derived from Rhizomucor miehei.

**3.** Process according to claim 1, wherein the lipase is derived from a modified Rhizomucor miehei, obtained by genetic engineering.

**4.** Process according to claims 1-3, wherein the enzyme is supported, preferably on an organic polymer.

**5.** Process according to claims 1-4, wherein the water activity is maintained between 0.01 and 0.4, in particular, between 0.05 and 0.3.

**6.** Process according to claims 1-5, wherein the overall degree of conversion is 50-85%.

**7.** Process according to claim 1, wherein the starting material contains triglycerides selected from the group consisting of: $S_3$; $U_3$; $O_3$; $M_3$; POO, StOO, StOSt; POP; BOB, $B_3$ or BOO (S= saturated fatty acid $C_2$-$C_{22}$; P= palmitic; St= stearic; O= oleic, M= $C_8$-$C_{14}$ fatty acid, B= behenic; U= unsaturated fatty acid).

**8.** Process according to claim 1, wherein the reactant is chosen from the group consisting of free fatty acids ($C_1$-$C_{26}$, preferably $C_2$-$C_{18}$); short chain alkyl esters thereof; $O_3$; $P_3$; $St_3$; or $B_3$, O = oleic acid; P = palmitic acid ; St = stearic acid and B = behenic acid residue.

**9.** Enzymically made asymmetric triglyceride compositions, characterised in that the molar ratio of the possible enantiomers is more than 1.5 or less than 0.7, preferably more than 3 or less than 0.33, in particular more than 4 or less than 0.25, and wherein the triglyceride is a composition rich in POSt/StOP, in particular comprising more than 40 wt% of chiral POSt/StOP, wherein P = palmitic ; St = stearic and O = oleic acid residue.

**10.** Chocolate-compositions, wherein at least part of the fat present consists of the enzymically made chiral triglyceride compositions according to claim 9.

## Patentansprüche

1. Verfahren zur Herstellung enzymatisch umgewandelter Triglyceride, dadurch gekennzeichnet, daß asymmetrische Triglyceride hergestellt werden, in denen verschiedene Stereoisomere vorhanden sind, wobei die Stereoisomere in einem anderen molekularen Verhältnis vorhanden sind als 1, wobei ein geeignetes Triglyceridausgangsmaterial mit einem geeigneten Reaktanten für die Einführung eines benötigten Fettsäurerests in das Ausgangstriglycerid umgewandelt wird, welches Verfahren unter Verwendung von Lipasen, die von Mucorspezies abgeleitet sind, auch bekannt als Zygomucor oder Rhizomucor, insbesondere Mucor miehei, durchgeführt wird, wobei die Wasseraktivität der Reaktionsmischung unterhalb 0,5 liegt und die Umwandlung unter Bedingungen durchgeführt wird, die einen Gesamtumwandlungsgrad von 40 bis 95% ergeben.

2. Verfahren nach Anspruch 1, in dem die Lipase von Rhizomucor miehei abgeleitet ist.

3. Verfahren nach Anspruch 1, in dem die Lipase von modifiziertem Rhizomucor miehei, erhalten durch gentechnische Verfahren, abgeleitet ist.

4. Verfahren nach den Ansprüchen 1 bis 3, in dem das Enzym auf einen Träger aufgebracht ist, bevorzugt auf ein organisches Polymer.

5. Verfahren nach den Ansprüchen 1 bis 4, in dem die Wasseraktivität zwischen 0,01 und 0,4, insbesondere zwischen 0,05 und 0,3 aufrechterhalten wird.

6. Verfahren nach den Ansprüchen 1 bis 5, in dem der Gesamtumwandlungsgrad 50 bis 85% beträgt.

7. Verfahren nach Anspruch 1, in dem das Ausgangsmaterial Triglyceride, ausgewählt aus der Gruppe, bestehend aus: $S_3$, $U_3$, $O_3$, $M_3$, POO, StOO, StOSt, POP, BOB, $B_3$ oder BOO (S=gesättigte $C_2$-$C_{22}$-Fettsäure; P=Palmitin-; St=Stearin-; O=Olein-; M=$C_8$-$C_{14}$-Fettsäure; B=Behen-; U=ungesättigte Fettsäure), enthält.

8. Verfahren nach Anspruch 1, in dem der Reaktant ausgewählt ist aus der Gruppe, bestehend aus freien Fettsäuren ($C_1$-$C_{26}$, bevorzugt $C_2$-$C_{18}$), kurzkettigen Alkylestern hiervon, $O_3$, $P_3$, $St_3$ oder $B_3$, O=Oleinsäure-, P=Palmitinsäure-, St=Stearinsäure- und B=Behensäurerest.

9. Enzymatisch hergestellte asymmetrische Triglyceridzusammensetzungen, dadurch gekennzeichnet, daß das Molverhältnis der möglichen Enantiomere mehr als 1,5 oder weniger als 0,7, bevorzugt mehr als 3 oder weniger als 0,33, insbesondere mehr als 4 oder weniger als 0,25, beträgt, und in denen das Triglycerid eine an POSt/StOP-reiche Zusammensetzung ist und insbesondere mehr als 40 Gew.-% an chiralem POSt/StOP umfaßt, wobei P=Palmitin-, St=Stearin- und O=Oleinsäurereste sind.

10. Schokoladenzusammensetzungen, in denen mindestens ein Teil des vorhandenen Fetts aus enzymatisch hergestellten chiralen Triglyceridzusammensetzungen nach Anspruch 9 besteht.

## Revendications

1. Procédé pour la préparation de triglycérides convertis par voie enzymatique, caractérisé en ce que l'on prépare des triglycérides asymétriques dans lesquels existent différents stéréoisomères, lesquels stéréoisomères sont présents en un rapport molaire différent de 1, un produit de départ triglycéridique approprié étant converti, avec un partenaire réactionnel approprié pour l'introduction d'un fragment acide gras requis dans le triglycéride de départ, lequel procédé est effectué à l'aide de lipases dérivées d'une espèce appartenant au genre *Mucor,* également connu sous le nom de *Zygomucor* ou *Rhizomucor,* en particulier *Mucor miehei,* tandis que l'activité de l'eau du mélange réactionnel est inférieure à 0,5, et la conversion est effectuée dans des conditions donnant un degré global de conversion de 40- 95 %.

2. Procédé selon la revendication 1, dans lequel la lipase est dérivée de *Rhizomucor miehei.*

3. Procédé selon la revendication 1, dans lequel la lipase est dérivée d'une souche modifiée de *Rhizomucor* miehei, obtenue par génie génétique.

**4.** Procédé selon les revendications 1 à 3, dans lequel l'enzyme est fixée sur un support, de préférence sur un polymère organique.

**5.** Procédé selon les revendications 1 à 4, dans lequel l'activité de l'eau est maintenue entre 0,01 et 0,4, en particulier entre 0,05 et 0,3.

**6.** Procédé selon les revendications à 5, dans lequel le degré global de conversion est de 50-85 %.

**7.** Procédé selon la revendication 1, dans lequel le produit de départ contient des triglycérides choisis dans le groupe constitué par: $S_3$, $U_3$, $O_3$, $M_3$, POO, StOO, StOSt, POP, BOB, $B_3$ et BOO (S = acide gras saturé en $C_2$-$C_{22}$, P = acide palmitique, St = acide stéarique, O = acide oléique, M = acide gras en $C_8$-$C_{14}$, B = acide béhénique, U = acide gras insaturé).

**8.** Procédé selon la revendication 1, dans lequel le partenaire réactionnel est choisi dans le groupe constitué par les acides gras libres (en $C_1$-$C_{26}$, de préférence en $C_2$-$C_{18}$), leurs esters alkyliques à chaîne courte, $O_3$, $P_3$, $St_3$ et $B_3$ (O = reste d'acide oléique, P = reste d'acide palmitique, St = reste d'acide stéarique et B = reste d'acide béhénique).

**9.** Compositions de triglycérides asymétriques obtenues par voie enzymatique, caractérisées en ce que le rapport molaire des énantiomères possibles est supérieur à 1,5 ou inférieur à 0,7, de préférence supérieur à 3 ou inférieur à 0,33, en particulier supérieur à 4 ou inférieur à 0,25 et dans lesquelles le triglyéride est une composition riche en POSt/StOP, en particulier comprenant plus de 40 % en poids de POSt/StOP chiral, P étant un reste d'acide palmitique, St étant un reste d'acide stéarique et O étant un reste d'acide oléique.

**10.** Compositions de chocolat dans lesquelles au moins une partie de la matière grasse présente consiste en les compositions de triglycérides chiraux obtenues par voie enzymatique selon la revendication 9.

Fig.1.

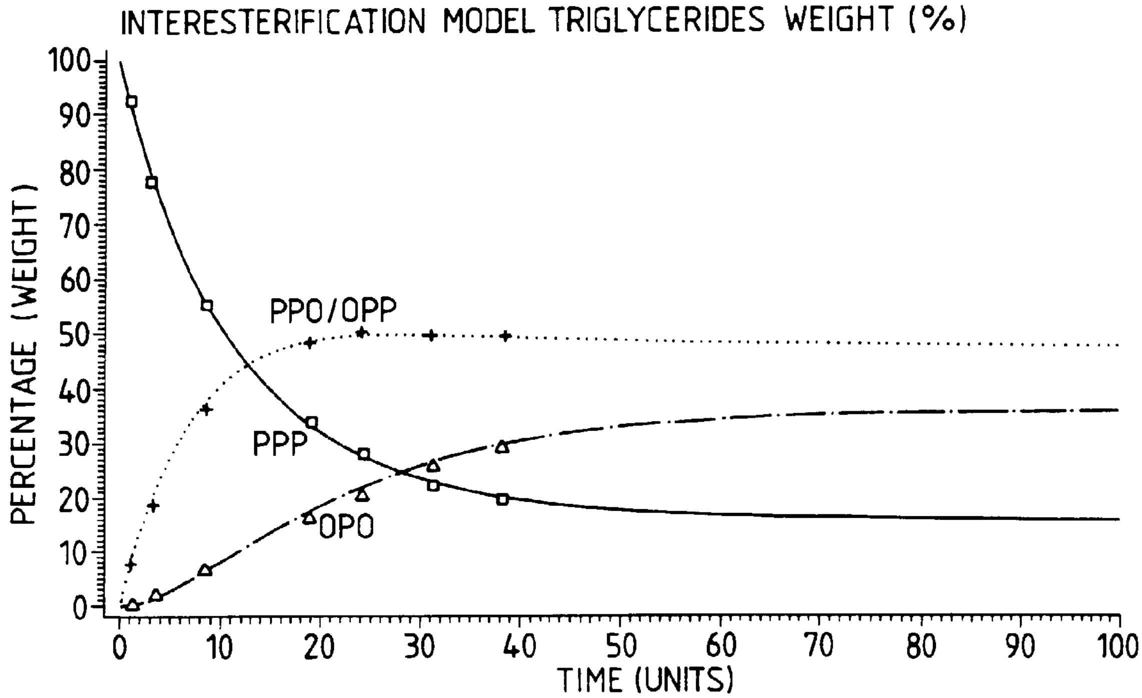


FINAL PERCENTAGES:PPP=16 PPO/OPP=48 OPO=36

Fig.2.

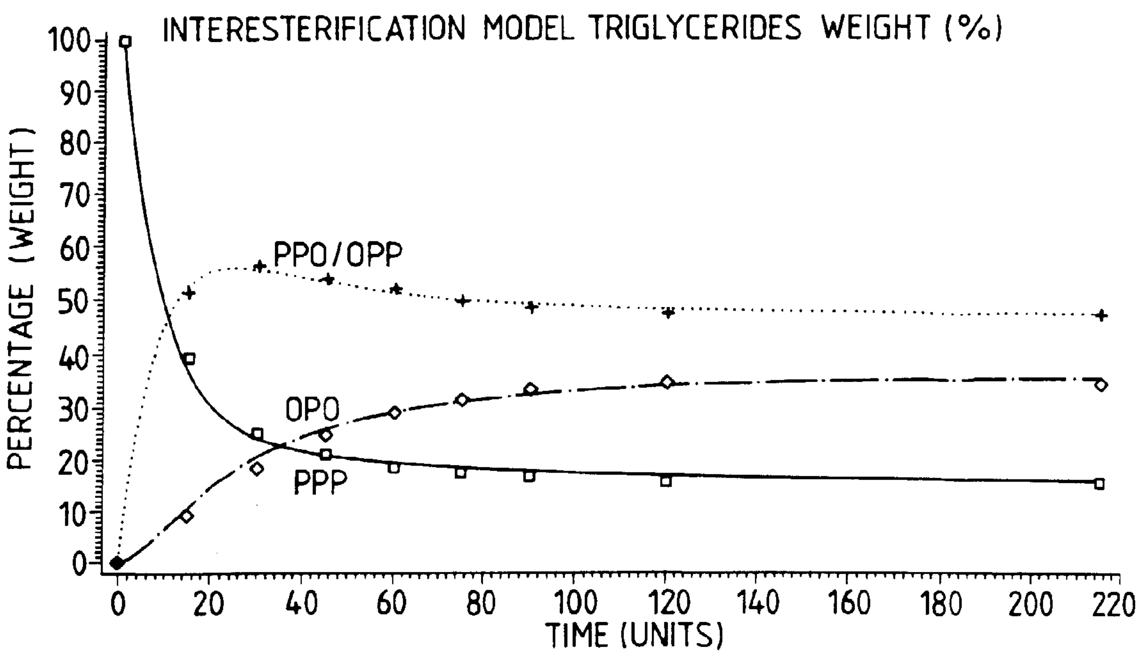


FIIIAL PERCENTAGES:PPP=16.1 PPO/OPP=48 OPO=35.9